# EUROPEAN PATENT SPECIFICATION

(11) **EP 2 083 073 B1**
(45) Date of publication and mention of the grant of the patent: **04.09.2013**
(21) Application number: 07768393.6
(22) Date of filing: 05.07.2007
(51) Int. Cl.: C07K 19/00, C12N 15/09

(54) **REAGENT CONTAINING FUSED PROTEIN OF SOLUBLE RANKL WITH EPITOPE TAG**
FUSIONSPROTEIN AUS LÖSLICHEM RANKL MIT EPITOP-TAG ENTHALTENDES REAGENS
REACTIF CONTENANT UNE PROTEINE FUSIONNEE DE RANKL SOLUBLE AVEC UN MARQUEUR D'EPITOPE

(30) Priority: 11.10.2006 JP 2006278052
(43) Date of publication of application: 29.07.2009
(73) Proprietor: Oriental Yeast Co., Ltd., Tokyo 1748505 (JP)
(72) Inventor: TOMIMORI, Yoshiya, Nagahama-shi Shiga 5260804 (JP); YASUDA, Hisataka, Nagahama-shi Shiga 5260804 (JP)
(74) Representative: HOFFMANN EITLE
(86) International application number: PCT/JP2007/063869
(87) International publication number: WO 2008/044378

(56) References cited:
- WO-A1-2006/068133
- JP-A- 2003 093 099
- JP-A- 2003 169 687
- JP-A- 2004 526 748
- JP-A- 2005 224 188
- LAM J ROSS F P TEITELBAUM L: "RANK ligand stimulates anabolic bone formation" ASBMR ANNUAL MEETING 23TH,, vol. 16, no. SUPPL. 1, 1 January 2001 (2001-01-01), XP003018734
- HOFBAUER L C ET AL: "RECEPTOR ACTIVATOR OF NUCLEAR FACTOR-KB LIGAND AND OSTEOPROTEGERIN. POTENTIAL IMPLICATIONS FOR THE PATHOGENESIS AND TREATMENT OF MALIGNANT BONE DISEASES" CANCER, AMERICAN CANCER SOCIETY, PHILADELPHIA, PA, US, vol. 92, no. 3, 1 August 2001 (2001-08-01) , pages 460-470, XP009071869 ISSN: 0008-543X
- XU J. ET AL.: 'Cloning, sequencing, and functional characterization of the rat homologue of receptor activator of NF-kappaB ligand' J. BONE MINER RES. vol. 15, no. 11, 2000, pages 2178 - 2186, XP003018735
- MEIYANTO E. ET AL.: 'Osteoclast differentiation factor modulates cell cycle machinery and causes a dela in s phase progression in RAW264 cells' BIOCHEM. BIOPHYS. RES. COMMUN. vol. 282, no. 1, 2001, pages 278 - 283, XP003018736
- KISHIMOTO Y. ET AL.: 'GST ga Yugo suru Koto ni yori RANKL no Kassei wa Zokyo sareru' THE JAPANESE SOCIETY FOR BONE AND MINERAL RESEARCH GAKUJUTSU SHUKAI PROGRAM SHOROKUSHU vol. 24TH, 2006, page 252, XP003018737
- MIZUNO A. ET AL.: 'Transgenic mice overexpressing soluble osteoclast differentiation factor (sODF) exhibit severe osteoporosis' J. BONE MINER. METAB. vol. 20, no. 6, 2002, pages 337 - 344, XP003018738
- KONG Y.Y. ET AL.: 'OPGL is a key regulator of osteoclastogenesis, lymphocyte development and lymph-node organogenesis' NATURE vol. 397, no. 6717, 1999, pages 315 - 323, XP003018739
- TOMIMORI Y. ET AL.: 'GST-RANKL Toyo ni yoru Kotsugensho-sho Model no Sakusei' DAI 8 KAI JAPAN OSTEOPOROSIS SOCIETY. HONE DOKKU.KENSHIN BUNKAKAI PROGRAM SHOROKUGO vol. 14, no. SUPPL. 1, 2006, page 139, XP003018740

### SEQUENCE LISTING

<110> ORIENTAL YEAST Co., Ltd.
<120> An agent comprising a fusion protein of a soluble RANKL and an epitope tag
<130> PH-3240-PCT
<150> JP 2006-278052
   <151> 2006-10-11
<160> 24
<170> PatentIn version 3.3
<210> 1
   <211> 2201
   <212> DNA
   <213> Homo sapiens
<220>
   <221> CDS
   <222> (129).. (1082)
<400> 1
   ggccaaagcc gggctccaag tcggcgcccc acgtcgaggc tccgccgcag cctccggagt     60
   tggccgcaga caagaagggg agggagcggg agagggagga gagctccgaa gcgagagggc    120
   cgagcgcc atg cgc cgc gcc agc aga gac tac acc aag tac ctg cgt ggc     170
            Met Arg Arg Ala Ser Arg Asp Tyr Thr Lys Tyr Leu Arg Gly
            1               5                   10
   tcg gag gag atg ggc ggc ggc ccc gga gcc ccg cac gag ggc ccc ctg      218
   Ser Glu Glu Met Gly Gly Gly Pro Gly Ala Pro His Glu Gly Pro Leu
   15                  20                  25                  30
   cac gcc ccg ccg ccg cct gcg ccg cac cag ccc ccc gcc gcc tcc cgc      266
   His Ala Pro Pro Pro Pro Ala Pro His Gln Pro Pro Ala Ala Ser Arg
                   35                  40                  45
   tcc atg ttc gtg gcc ctc ctg ggg ctg ggg ctg ggc cag gtt gtc tgc      314
   Ser Met Phe Val Ala Leu Leu Gly Leu Gly Leu Gly Gln Val Val Cys
                   50                  55                  60
   agc gtc gcc ctg ttc ttc tat ttc aga gcg cag atg gat cct aat aga      362
   Ser Val Ala Leu Phe Phe Tyr Phe Arg Ala Gln Met Asp Pro Asn Arg
               65                  70                  75
   ata tca gaa gat ggc act cac tgc att tat aga att ttg aga ctc cat      410
   Ile Ser Glu Asp Gly Thr His Cys Ile Tyr Arg Ile Leu Arg Leu His
       80                  85                  90
   gaa aat gca gat ttt caa gac aca act ctg gag agt caa gat aca aaa      458
   Glu Asn Ala Asp Phe Gln Asp Thr Thr Leu Glu Ser Gln Asp Thr Lys
   95                  100                 105                 110
   tta ata cct gat tca tgt agg aga att aaa cag gcc ttt caa gga gct      506
   Leu Ile Pro Asp Ser Cys Arg Arg Ile Lys Gln Ala Phe Gln Gly Ala
                   115                 120                 125
   gtg caa aag gaa tta caa cat atc gtt gga tca cag cac atc aga gca      554
   Val Gln Lys Glu Leu Gln His Ile Val Gly Ser Gln His Ile Arg Ala
               130                 135                 140
   gag aaa gcg atg gtg gat ggc tca tgg tta gat ctg gcc aag agg agc      602
   Glu Lys Ala Met Val Asp Gly Ser Trp Leu Asp Leu Ala Lys Arg Ser
           145                 150                 155
   aag ctt gaa gct cag cct ttt gct cat ctc act att aat gcc acc gac      650
   Lys Leu Glu Ala Gln Pro Phe Ala His Leu Thr Ile Asn Ala Thr Asp
       160                 165                 170
   atc cca tct ggt tcc cat aaa gtg agt ctg tcc tct tgg tac cat gat      698
   Ile Pro Ser Gly Ser His Lys Val Ser Leu Ser Ser Trp Tyr His Asp
   175                 180                 185                 190
   cgg ggt tgg gcc aag atc tcc aac atg act ttt agc aat gga aaa cta      746
   Arg Gly Trp Ala Lys Ile Ser Asn Met Thr Phe Ser Asn Gly Lys Leu
                   195                 200                 205
   ata gtt aat cag gat ggc ttt tat tac ctg tat gcc aac att tgc ttt      794
   Ile Val Asn Gln Asp Gly Phe Tyr Tyr Leu Tyr Ala Asn Ile Cys Phe
   210 215 220
   cga cat cat gaa act tca gga gac cta gct aca gag tat ctt caa cta 842
   Arg His His Glu Thr Ser Gly Asp Leu Ala Thr Glu Tyr Leu Gln Leu
   225 230 235
   atg gtg tac gtc act aaa acc agc atc aaa atc cca agt tct cat acc 890
   Met Val Tyr Val Thr Lys Thr Ser Ile Lys Ile Pro Ser Ser His Thr
   240 245 250
   ctg atg aaa gga gga agc acc aag tat tgg tca ggg aat tct gaa ttc 938
   Leu Met Lys Gly Gly Ser Thr Lys Tyr Trp Ser Gly Asn Ser Glu Phe
   255 260 265 270
   cat ttt tat tcc ata aac gtt ggt gga ttt ttt aag tta cgg tct gga 986
   His Phe Tyr Ser Ile Asn Val Gly Gly Phe Phe Lys Leu Arg Ser Gly
   275 280 285
   gag gaa atc agc atc gag gtc tcc aac ccc tcc tta ctg gat ccg gat 1034
   Glu Glu Ile Ser Ile Glu Val Ser Asn Pro Ser Leu Leu Asp Pro Asp
   290 295 300
   cag gat gca aca tac ttt ggg gct ttt aaa gtt cga gat ata gat tga 1082
   Gln Asp Ala Thr Tyr Phe Gly Ala Phe Lys Val Arg Asp Ile Asp
   305 310 315
   gccccagttt ttggagtgtt atgtatttcc tggatgtttg gaaacatttt ttaaaacaag 1142
   ccaagaaaga tgtatatagg tgtgtgagac tactaagagg catggcccca acggtacacg 1202
   actcagtatc catgctcttg accttgtaga gaacacgcgt atttacctgc cagtgggaga 1262
   tgttagactc atggtgtgtt acacaatggt ttttaaattt tgtaatgaat tcctagaatt 1322
   aaaccagatt ggagcaatta cgggttgacc ttatgagaaa ctgcatgtgg gctatgggag 1382
   gggttggtcc ctggtcatgt gccccttcgc agctgaagtg gagagggtgt catctagcgc 1442
   aattgaagga tcatctgaag ggcaaattcc ttttgaattg ttacatcatg ctggaacctg 1502
   caaaaaatac tttttctaat gaggagagaa aatatatgta tttttatata atatctaaag 1562
   ttatatttca gatgtaatgt tttctttgca aagtattgta aattatattt gtgctatagt 1622
   atttgattca aaatatttaa aaatgtcttg ctgttgacat atttaatgtt ttaaatgtac 1682
   agacatattt aactggtgca ctttgtaaat tccctgggga aaacttgcag ctaaggaggg 1742
   gaaaaaaatg ttgtttccta atatcaaatg cagtatattt cttcgttctt tttaagttaa 1802
   tagatttttt cagacttgtc aagcctgtgc aaaaaaatta aaatggatgc cttgaataat 1862
   aagcaggatg ttggccacca ggtgcctttc aaatttagaa actaattgac tttagaaagc 1922
   tgacattgcc aaaaaggata cataatgggc cactgaaatt tgtcaagagt agttatataa 1982
   ttgttgaaca ggtgtttttc cacaagtgcc gcaaattgta cctttttttt tttttcaaaa 2042
   tagaaaagtt attagtggtt tatcagcaaa aaagtccaat tttaatttag taaatgttat 2102
   tttatactgt acaataaaaa cattgccttt gaatgttaat tttttggtac aaaaataaat 2162
   ttatatgaaa aaaaaaaaaa aaaaaaaaaa aaaaaaaaa 2201
<210> 2
   <211> 317
   <212> PRT
   <213> Homo sapiens
<400> 2
   Met Arg Arg Ala Ser Arg Asp Tyr Thr Lys Tyr Leu Arg Gly Ser Glu
   1 5 10 15
   Glu Met Gly Gly Gly Pro Gly Ala Pro His Glu Gly Pro Leu His Ala
   20 25 30
   Pro Pro Pro Pro Ala Pro His Gln Pro Pro Ala Ala Ser Arg Ser Met
   35 40 45
   Phe Val Ala Leu Leu Gly Leu Gly Leu Gly Gln Val Val Cys Ser Val
   50 55 60
   Ala Leu Phe Phe Tyr Phe Arg Ala Gln Met Asp Pro Asn Arg Ile Ser
   65 70 75 80
   Glu Asp Gly Thr His Cys Ile Tyr Arg Ile Leu Arg Leu His Glu Asn
   85 90 95
   Ala Asp Phe Gln Asp Thr Thr Leu Glu Ser Gln Asp Thr Lys Leu Ile
   100 105 110
   Pro Asp Ser Cys Arg Arg Ile Lys Gln Ala Phe Gln Gly Ala Val Gln
   115 120 125
   Lys Glu Leu Gln His Ile Val Gly Ser Gln His Ile Arg Ala Glu Lys
   130 135 140
   Ala Met Val Asp Gly Ser Trp Leu Asp Leu Ala Lys Arg Ser Lys Leu
   145 150 155 160
   Glu Ala Gln Pro Phe Ala His Leu Thr Ile Asn Ala Thr Asp Ile Pro
   165 170 175
   Ser Gly Ser His Lys Val Ser Leu Ser Ser Trp Tyr His Asp Arg Gly
   180 185 190
   Trp Ala Lys Ile Ser Asn Met Thr Phe Ser Asn Gly Lys Leu Ile Val
   195 200 205
   Asn Gln Asp Gly Phe Tyr Tyr Leu Tyr Ala Asn Ile Cys Phe Arg His
   210 215 220
   His Glu Thr Ser Gly Asp Leu Ala Thr Glu Tyr Leu Gln Leu Met Val
   225 230 235 240
   Tyr Val Thr Lys Thr Ser Ile Lys Ile Pro Ser Ser His Thr Leu Met
   245 250 255
   Lys Gly Gly Ser Thr Lys Tyr Trp Ser Gly Asn Ser Glu Phe His Phe
   260 265 270
   Tyr Ser Ile Asn Val Gly Gly Phe Phe Lys Leu Arg Ser Gly Glu Glu
   275 280 285
   Ile Ser Ile Glu Val Ser Asn Pro Ser Leu Leu Asp Pro Asp Gln Asp
   290 295 300
   Ala Thr Tyr Phe Gly Ala Phe Lys Val Arg Asp Ile Asp
   305 310 315
<210> 3
   <211> 8
   <212> PRT
   <213> Artificial
<220>
   <223> Synthetic
<400> 3
   Asp Tyr Lys Asp Asp Asp Asp Lys
   1 5
<210> 4
   <211> 10
   <212> PRT
   <213> Artificial
<220>
   <223> Synthetic
<400> 4
   Glu Gln Lys Leu Ile Ser Glu Glu Asp Leu
   1 5 10
<210> 5
   <211> 14
   <212> PRT
   <213> Artificial
<220>
   <223> Synthetic
<400> 5
   Gly Lys Pro Ile Pro Asn Pro Leu Leu Gly Leu Asp Ser Thr
   1 5 10
<210> 6
   <211> 6
   <212> PRT
   <213> Artificial
<220>
   <223> Synthetic
<400> 6
   His Gln His Gln His Gln
   1 5
<210> 7
   <211> 9
   <212> PRT
   <213> Artificial
<220>
   <223> Synthetic
<400> 7
   Tyr Pro Tyr Asp Val Pro Asp Tyr Ala
   1 5
<210> 8
   <211> 6
   <212> PRT
   <213> Artificial
<220>
   <223> Synthetic
<400> 8
   Asp Thr Tyr Arg Tyr Ile
   1 5
<210> 9
   <211> 11
   <212> PRT
   <213> Artificial
<220>
   <223> Synthetic
<400> 9
   Met Ala Ser Met Thr Gly Gly Gln Gln Met Gly
   1 5 10
<210> 10
   <211> 11
   <212> PRT
   <213> Artificial
<220>
   <223> Synthetic
<400> 10
   Tyr Thr Asp Ile Glu Met Asn Arg Leu Gly Lys
   1 5 10
<210> 11
   <211> 5
   <212> PRT
   <213> Artificial
<220>
   <223> Synthetic
<400> 11
   Asp Asp Asp Asp Lys
   1 5
<210> 12
   <211> 16
   <212> PRT
   <213> Artificial
<220>
   <223> Synthetic
<400> 12
   Lys Glu Thr Ala Ala Ala Lys Phe Glu Arg Gln His Ile Asp Ser Cys
   1 5 10 15
<210> 13
   <211> 12
   <212> PRT
   <213> Artificial
<220>
   <223> Synthetic
<400> 13
   Met Lys Ala Glu Phe Arg Arg Gln Glu Ser Asp Arg
   1 5 10
<210> 14
   <211> 12
   <212> PRT
   <213> Artificial
<220>
   <223> Synthetic
<400> 14
   Met Arg Asp Ala Leu Asp Arg Leu Asp Arg Leu Ala
   1 5 10
<210> 15
   <211> 12
   <212> PRT
   <213> Artificial
<220>
   <223> Synthetic
<400> 15
   Met Lys Asp Gly Glu Glu Tyr Ser Arg Ala Phe Arg
   1 5 10
<210> 16
   <211> 9
   <212> PRT
   <213> Artificial
<220>
   <223> Synthetic
<400> 16
   Glu Glu Glu Glu Tyr Met Pro Met Glu
   1 5
<210> 17
   <211> 12
   <212> PRT
   <213> Artificial
<220>
   <223> Synthetic
<400> 17
   Cys Thr Pro Thr Asp Val Pro Asp Tyr Ala Ser Leu
   1 5 10
<210> 18
   <211> 6
   <212> PRT
   <213> Artificial
<220>
   <223> Synthetic
<400> 18
   Pro Pro Glu Pro Glu Thr
   1 5
<210> 19
   <211> 1275
   <212> DNA
   <213> Artificial
<220>
   <223> GST-RANKL (aa127-317)
<400> 19
   atgtccccta tactaggtta ttggaaaatt aagggccttg tgcaacccac tcgacttctt 60
   ttggaatatc ttgaagaaaa atatgaagag catttgtatg agcgcgatga aggtgataaa 120
   tggcgaaaca aaaagtttga attgggtttg gagtttccca atcttcctta ttatattgat 180
   ggtgatgtta aattaacaca gtctatggcc atcatacgtt atatagctga caagcacaac 240
   atgttgggtg gttgtccaaa agagcgtgca gagatttcaa tgcttgaagg agcggttttg 300
   gatattagat acggtgtttc gagaattgca tatagtaaag actttgaaac tctcaaagtt 360
   gattttctta gcaagctacc tgaaatgctg aaaatgttcg aagatcgttt atgtcataaa 420
   acatatttaa atggtgatca tgtaacccat cctgacttca tgttgtatga cgctcttgat 480
   gttgttttat acatggaccc aatgtgcctg gatgcgttcc caaaattagt ttgttttaaa 540
   aaacgtattg aagctatccc acaaattgat aagtacttga aatccagcaa gtatatagca 600
   tggcctttgc agggctggca agccacgttt ggtggtggcg accatcctcc aaaatcggat 660
   ctggttccgc gtggatcccc aggaattccc gggtcgactg tgcaaaagga attacaacat 720
   atcgttggat cacagcacat cagagcagag aaagcgatgg tggatggctc atggttagat 780
   ctggccaaga ggagcaagct tgaagctcag ccttttgctc atctcactat taatgccacc 840
   gacatcccat ctggttccca taaagtgagt ctgtcctctt ggtaccatga tcggggttgg 900
   gccaagatct ccaacatgac ttttagcaat ggaaaactaa tagttaatca ggatggcttt 960
   tattacctgt atgccaacat ttgctttcga catcatgaaa cttcaggaga cctagctaca 1020
   gagtatcttc aactaatggt gtacgtcact aaaaccagca tcaaaatccc aagttctcat 1080
   accctgatga aaggaggaag caccaagtat tggtcaggga attctgaatt ccatttttat 1140
   tccataaacg ttggtggatt ttttaagtta cggtctggag aggaaatcag catcgaggtc 1200
   tccaacccct ccttactgga tccggatcag gatgcaacat actttggggc ttttaaagtt 1260
   cgagatatag attga 1275
<210> 20
   <211> 442
   <212> PRT
   <213> Artificial
<220>
   <223> GST-RANKL (aa127-317)
<400> 20
   Met Ser Pro Ile Leu Gly Tyr Trp Lys Ile Lys Gly Leu Val Gln Pro
   1 5 10 15
   Thr Arg Leu Leu Leu Glu Tyr Leu Glu Glu Lys Tyr Glu Glu His Leu
   20 25 30
   Tyr Glu Arg Asp Glu Gly Asp Lys Trp Arg Asn Lys Lys Phe Glu Leu
   35 40 45
   Gly Leu Glu Phe Pro Asn Leu Pro Tyr Tyr Ile Asp Gly Asp Val Lys
   50 55 60
   Leu Thr Gln Ser Met Ala Ile Ile Arg Tyr Ile Ala Asp Lys His Asn
   65 70 75 80
   Met Leu Gly Gly Cys Pro Lys Glu Arg Ala Glu Ile Ser Met Leu Glu
   85 90 95
   Gly Ala Val Leu Asp Ile Arg Tyr Gly Val Ser Arg Ile Ala Tyr Ser
   100 105 110
   Lys Asp Phe Glu Thr Leu Lys Val Asp Phe Leu Ser Lys Leu Pro Glu
   115 120 125
   Met Leu Lys Met Phe Glu Asp Arg Leu Cys His Lys Thr Tyr Leu Asn
   130 135 140
   Gly Asp His Val Thr His Pro Asp Phe Met Leu Tyr Asp Ala Leu Asp
   145 150 155 160
   Val Val Leu Tyr Met Asp Pro Met Cys Leu Asp Ala Phe Pro Lys Leu
   165 170 175
   Val Cys Phe Lys Lys Arg Ile Glu Ala Ile Pro Gln Ile Asp Lys Tyr
   180 185 190
   Leu Lys Ser Ser Lys Tyr Ile Ala Trp Pro Leu Gln Gly Trp Gln Ala
   195 200 205
   Thr Phe Gly Gly Gly Asp His Pro Pro Lys Ser Asp Leu Val Pro Arg
   210 215 220
   Gly Ser Pro Gly Ile Pro Gly Ser Thr Arg Ala Ala Ala Ser Leu Val
   225 230 235 240
   Pro Arg Gly Ser Pro Gly Ile Pro Gly Ser Thr Val Gln Lys Glu Leu
   245 250 255
   Gln His Ile Val Gly Ser Gln His Ile Arg Ala Glu Lys Ala Met Val
   260 265 270
   Asp Gly Ser Trp Leu Asp Leu Ala Lys Arg Ser Lys Leu Glu Ala Gln
   275 280 285
   Pro Phe Ala His Leu Thr Ile Asn Ala Thr Asp Ile Pro Ser Gly Ser
   290 295 300
   His Lys Val Ser Leu Ser Ser Trp Tyr His Asp Arg Gly Trp Ala Lys
   305 310 315 320
   Ile Ser Asn Met Thr Phe Ser Asn Gly Lys Leu Ile Val Asn Gln Asp
   325 330 335
   Gly Phe Tyr Tyr Leu Tyr Ala Asn Ile Cys Phe Arg His His Glu Thr
   340 345 350
   Ser Gly Asp Leu Ala Thr Glu Tyr Leu Gln Leu Met Val Tyr Val Thr
   355 360 365
   Lys Thr Ser Ile Lys Ile Pro Ser Ser His Thr Leu Met Lys Gly Gly
   370 375 380
   Ser Thr Lys Tyr Trp Ser Gly Asn Ser Glu Phe His Phe Tyr Ser Ile
   385 390 395 400
   Asn Val Gly Gly Phe Phe Lys Leu Arg Ser Gly Glu Glu Ile Ser Ile
   405 410 415
   Glu Val Ser Asn Pro Ser Leu Leu Asp Pro Asp Gln Asp Ala Thr Tyr
   420 425 430
   Phe Gly Ala Phe Lys Val Arg Asp Ile Asp
   435 440
<210> 21
   <211> 1236
   <212> DNA
   <213> Artificial
<220>
   <223> GST-RANKL (aa140-317)
<400> 21
   atgtccccta tactaggtta ttggaaaatt aagggccttg tgcaacccac tcgacttctt 60
   ttggaatatc ttgaagaaaa atatgaagag catttgtatg agcgcgatga aggtgataaa 120
   tggcgaaaca aaaagtttga attgggtttg gagtttccca atcttcctta ttatattgat 180
   ggtgatgtta aattaacaca gtctatggcc atcatacgtt atatagctga caagcacaac 240
   atgttgggtg gttgtccaaa agagcgtgca gagatttcaa tgcttgaagg agcggttttg 300
   gatattagat acggtgtttc gagaattgca tatagtaaag actttgaaac tctcaaagtt 360
   gattttctta gcaagctacc tgaaatgctg aaaatgttcg aagatcgttt atgtcataaa 420
   acatatttaa atggtgatca tgtaacccat cctgacttca tgttgtatga cgctcttgat 480
   gttgttttat acatggaccc aatgtgcctg gatgcgttcc caaaattagt ttgttttaaa 540
   aaacgtattg aagctatccc acaaattgat aagtacttga aatccagcaa gtatatagca 600
   tggcctttgc agggctggca agccacgttt ggtggtggcg accatcctcc aaaatcggat 660
   ctggttccgc gtggatcccc aggaattccc gggtcgacta tcagagcaga gaaagcgatg 720
   gtggatggct catggttaga tctggccaag aggagcaagc ttgaagctca gccttttgct 780
   catctcacta ttaatgccac cgacatccca tctggttccc ataaagtgag tctgtcctct 840
   tggtaccatg atcggggttg ggccaagatc tccaacatga cttttagcaa tggaaaacta 900
   atagttaatc aggatggctt ttattacctg tatgccaaca tttgctttcg acatcatgaa 960
   acttcaggag acctagctac agagtatctt caactaatgg tgtacgtcac taaaaccagc 1020
   atcaaaatcc caagttctca taccctgatg aaaggaggaa gcaccaagta ttggtcaggg 1080
   aattctgaat tccattttta ttccataaac gttggtggat tttttaagtt acggtctgga 1140
   gaggaaatca gcatcgaggt ctccaacccc tccttactgg atccggatca ggatgcaaca 1200
   tactttgggg cttttaaagt tcgagatata gattga 1236
<210> 22
   <211> 429
   <212> PRT
   <213> Artificial
<220>
   <223> GST-RANKL (aa140-317)
<400> 22
   Met Ser Pro Ile Leu Gly Tyr Trp Lys Ile Lys Gly Leu Val Gln Pro
   1 5 10 15
   Thr Arg Leu Leu Leu Glu Tyr Leu Glu Glu Lys Tyr Glu Glu His Leu
   20 25 30
   Tyr Glu Arg Asp Glu Gly Asp Lys Trp Arg Asn Lys Lys Phe Glu Leu
   35 40 45
   Gly Leu Glu Phe Pro Asn Leu Pro Tyr Tyr Ile Asp Gly Asp Val Lys
   50 55 60
   Leu Thr Gln Ser Met Ala Ile Ile Arg Tyr Ile Ala Asp Lys His Asn
   65 70 75 80
   Met Leu Gly Gly Cys Pro Lys Glu Arg Ala Glu Ile Ser Met Leu Glu
   85 90 95
   Gly Ala Val Leu Asp Ile Arg Tyr Gly Val Ser Arg Ile Ala Tyr Ser
   100 105 110
   Lys Asp Phe Glu Thr Leu Lys Val Asp Phe Leu Ser Lys Leu Pro Glu
   115 120 125
   Met Leu Lys Met Phe Glu Asp Arg Leu Cys His Lys Thr Tyr Leu Asn
   130 135 140
   Gly Asp His Val Thr His Pro Asp Phe Met Leu Tyr Asp Ala Leu Asp
   145 150 155 160
   Val Val Leu Tyr Met Asp Pro Met Cys Leu Asp Ala Phe Pro Lys Leu
   165 170 175
   Val Cys Phe Lys Lys Arg Ile Glu Ala Ile Pro Gln Ile Asp Lys Tyr
   180 185 190
   Leu Lys Ser Ser Lys Tyr Ile Ala Trp Pro Leu Gln Gly Trp Gln Ala
   195 200 205
   Thr Phe Gly Gly Gly Asp His Pro Pro Lys Ser Asp Leu Val Pro Arg
   210 215 220
   Gly Ser Pro Gly Ile Pro Gly Ser Thr Arg Ala Ala Ala Ser Leu Val
   225 230 235 240
   Pro Arg Gly Ser Pro Gly Ile Pro Gly Ser Thr Ile Arg Ala Glu Lys
   245 250 255
   Ala Met Val Asp Gly Ser Trp Leu Asp Leu Ala Lys Arg Ser Lys Leu
   260 265 270
   Glu Ala Gln Pro Phe Ala His Leu Thr Ile Asn Ala Thr Asp Ile Pro
   275 280 285
   Ser Gly Ser His Lys Val Ser Leu Ser Ser Trp Tyr His Asp Arg Gly
   290 295 300
   Trp Ala Lys Ile Ser Asn Met Thr Phe Ser Asn Gly Lys Leu Ile Val
   305 310 315 320
   Asn Gln Asp Gly Phe Tyr Tyr Leu Tyr Ala Asn Ile Cys Phe Arg His
   325 330 335
   His Glu Thr Ser Gly Asp Leu Ala Thr Glu Tyr Leu Gln Leu Met Val
   340 345 350
   Tyr Val Thr Lys Thr Ser Ile Lys Ile Pro Ser Ser His Thr Leu Met
   355 360 365
   Lys Gly Gly Ser Thr Lys Tyr Trp Ser Gly Asn Ser Glu Phe His Phe
   370 375 380
   Tyr Ser Ile Asn Val Gly Gly Phe Phe Lys Leu Arg Ser Gly Glu Glu
   385 390 395 400
   Ile Ser Ile Glu Val Ser Asn Pro Ser Leu Leu Asp Pro Asp Gln Asp
   405 410 415
   Ala Thr Tyr Phe Gly Ala Phe Lys Val Arg Asp Ile Asp
   420 425
<210> 23
   <211> 1179
   <212> DNA
   <213> Artificial
<220>
   <223> GST-RANKL (aa159-317)
<400> 23
   atgtccccta tactaggtta ttggaaaatt aagggccttg tgcaacccac tcgacttctt 60
   ttggaatatc ttgaagaaaa atatgaagag catttgtatg agcgcgatga aggtgataaa 120
   tggcgaaaca aaaagtttga attgggtttg gagtttccca atcttcctta ttatattgat 180
   ggtgatgtta aattaacaca gtctatggcc atcatacgtt atatagctga caagcacaac 240
   atgttgggtg gttgtccaaa agagcgtgca gagatttcaa tgcttgaagg agcggttttg 300
   gatattagat acggtgtttc gagaattgca tatagtaaag actttgaaac tctcaaagtt 360
   gattttctta gcaagctacc tgaaatgctg aaaatgttcg aagatcgttt atgtcataaa 420
   acatatttaa atggtgatca tgtaacccat cctgacttca tgttgtatga cgctcttgat 480
   gttgttttat acatggaccc aatgtgcctg gatgcgttcc caaaattagt ttgttttaaa 540
   aaacgtattg aagctatccc acaaattgat aagtacttga aatccagcaa gtatatagca 600
   tggcctttgc agggctggca agccacgttt ggtggtggcg accatcctcc aaaatcggat 660
   ctggttccgc gtggatcccc aggaattccc gggtcgacta agcttgaagc tcagcctttt 720
   gctcatctca ctattaatgc caccgacatc ccatctggtt cccataaagt gagtctgtcc 780
   tcttggtacc atgatcgggg ttgggccaag atctccaaca tgacttttag caatggaaaa 840
   ctaatagtta atcaggatgg cttttattac ctgtatgcca acatttgctt tcgacatcat 900
   gaaacttcag gagacctagc tacagagtat cttcaactaa tggtgtacgt cactaaaacc 960
   agcatcaaaa tcccaagttc tcataccctg atgaaaggag gaagcaccaa gtattggtca 1020
   gggaattctg aattccattt ttattccata aacgttggtg gattttttaa gttacggtct 1080
   ggagaggaaa tcagcatcga ggtctccaac ccctccttac tggatccgga tcaggatgca 1140
   acatactttg gggcttttaa agttcgagat atagattga 1179
<210> 24
   <211> 410
   <212> PRT
   <213> Artificial
<220>
   <223> GST-RANKL (aa159-317)
<400> 24
   Met Ser Pro Ile Leu Gly Tyr Trp Lys Ile Lys Gly Leu Val Gln Pro
   1 5 10 15
   Thr Arg Leu Leu Leu Glu Tyr Leu Glu Glu Lys Tyr Glu Glu His Leu
   20 25 30
   Tyr Glu Arg Asp Glu Gly Asp Lys Trp Arg Asn Lys Lys Phe Glu Leu
   35 40 45
   Gly Leu Glu Phe Pro Asn Leu Pro Tyr Tyr Ile Asp Gly Asp Val Lys
   50 55 60
   Leu Thr Gln Ser Met Ala Ile Ile Arg Tyr Ile Ala Asp Lys His Asn
   65 70 75 80
   Met Leu Gly Gly Cys Pro Lys Glu Arg Ala Glu Ile Ser Met Leu Glu
   85 90 95
   Gly Ala Val Leu Asp Ile Arg Tyr Gly Val Ser Arg Ile Ala Tyr Ser
   100 105 110
   Lys Asp Phe Glu Thr Leu Lys Val Asp Phe Leu Ser Lys Leu Pro Glu
   115 120 125
   Met Leu Lys Met Phe Glu Asp Arg Leu Cys His Lys Thr Tyr Leu Asn
   130 135 140
   Gly Asp His Val Thr His Pro Asp Phe Met Leu Tyr Asp Ala Leu Asp
   145 150 155 160
   Val Val Leu Tyr Met Asp Pro Met Cys Leu Asp Ala Phe Pro Lys Leu
   165 170 175
   Val Cys Phe Lys Lys Arg Ile Glu Ala Ile Pro Gln Ile Asp Lys Tyr
   180 185 190
   Leu Lys Ser Ser Lys Tyr Ile Ala Trp Pro Leu Gln Gly Trp Gln Ala
   195 200 205
   Thr Phe Gly Gly Gly Asp His Pro Pro Lys Ser Asp Leu Val Pro Arg
   210 215 220
   Gly Ser Pro Gly Ile Pro Gly Ser Thr Arg Ala Ala Ala Ser Leu Val
   225 230 235 240
   Pro Arg Gly Ser Pro Gly Ile Pro Gly Ser Thr Lys Leu Glu Ala Gln
   245 250 255
   Pro Phe Ala His Leu Thr Ile Asn Ala Thr Asp Ile Pro Ser Gly Ser
   260 265 270
   His Lys Val Ser Leu Ser Ser Trp Tyr His Asp Arg Gly Trp Ala Lys
   275 280 285
   Ile Ser Asn Met Thr Phe Ser Asn Gly Lys Leu Ile Val Asn Gln Asp
   290 295 300
   Gly Phe Tyr Tyr Leu Tyr Ala Asn Ile Cys Phe Arg His His Glu Thr
   305 310 315 320
   Ser Gly Asp Leu Ala Thr Glu Tyr Leu Gln Leu Met Val Tyr Val Thr
   325 330 335
   Lys Thr Ser Ile Lys Ile Pro Ser Ser His Thr Leu Met Lys Gly Gly
   340 345 350
   Ser Thr Lys Tyr Trp Ser Gly Asn Ser Glu Phe His Phe Tyr Ser Ile
   355 360 365
   Asn Val Gly Gly Phe Phe Lys Leu Arg Ser Gly Glu Glu Ile Ser Ile
   370 375 380
   Glu Val Ser Asn Pro Ser Leu Leu Asp Pro Asp Gln Asp Ala Thr Tyr
   385 390 395 400
   Phe Gly Ala Phe Lys Val Arg Asp Ile Asp
   405 410

## Description

### Technical Field

The present invention relates to the use of an agent containing a fused protein of soluble RANKL with a (GST (glutathione-S-transferase) epitope tag, such protein being capable of inducing osteoclast differentiation and activation.

### Background Art

Osteoclasts, which control osteolysis, are large multinucleated cells derived from hematopoietic cells that differentiate into monocytes/macrophages. Differentiation and maturation of osteoclast precursor cells into osteoclasts are controlled by osteoblasts/stromal cells on the bone surface. An osteoclast differentiation factor (RANKL; receptor activator of NF-κB ligand) is a membrane-bound protein belonging to the family of tumor necrosis factors (TNFs) guided by bone resorption factors onto osteoblasts/stromal cells, and it is essential for osteoclast differentiation and maturation (Non-Patent Documents 1 and 2). It has been known that RANKL is partially cleaved by metalloprotease in an extracellular region so as to result in soluble RANKL. In practice, soluble RANKL is known to induce *in vitro* differentiation of macrophage precursor cells into osteoclasts when coexisting with M-CSF. However, it cannot be said that soluble RANKL products, including those that are commercially available, have strong levels of bioactivity. Therefore, even when osteoclasts are formed by cell culture with the use of myelocytes, spleen cells, precursor cells in the peripheral blood, a macrophage cell line, or the like. a number of very large osteoclasts cannot be readily obtained.

Meanwhile, in order to increase the amount of recombinant protein produced or to facilitate recombinant protein purification, a variety of proteins and peptides have been used, which can also be used as tags. Such proteins and peptides include glutathione-S-transferase (GST) (see Non-Patent Document 3), histidine tag (His tag) / thioredoxin (TRX) (see Patent Documents 1 and 2 and Non-Patent Documents 4 and 5), flag tag (FLAG) (see Patent Documents 3 and 4), Myc tag (see Non-Patent Document 6), V5 tag (see Non-Patent Document 7), Xpress tag, and an immunoglobulin Fc region. These proteins and peptides have been used for the above purposes; however, they have not been used for the purpose of increasing the activity of fused protein.
Patent Document 1: US Patent No. 5270181
Patent Document 2: US Patent No. 5292646
Patent Document 3: JP Patent No. 1983150
Patent Document 4: JP Patent No. 2665359
Non-Patent Document 1: Yasuda et al., Proc Natl Acad Sci USA 95: 3597, 1998
Non-Patent Document 2: Lacey et al., Cell 93: 165, 1998
Non-Patent Document 3: Kaelin et al., Cell 70: 351, 1992
Non-Patent Document 4: La Vallie et al., Bio/Technology 11: 187, 1993
Non-Patent Document 5: Lu et al., J Biol Chem 271: 5059, 1996
Non-Patent Document 6: Evans et al., Mol Cell Biol 5, 3610, 1985
Non-Patent Document 7: Southern et al., J Gen Virol 72, 1551, 1991

Lam J et al ("RANK ligand stimulates anabolic bone formation" ASBMR ANNUAL MEETING 23TH,, vol. 16, no. SUPPL. 1, 1 January 2001) state that murine RANKL when administered as an amino-terminal GST fusion protein profoundly stimulates anabolic bone formation *in vitro, ex vivo* and *in vivo,* but fails to affect the osteoclast number. They suggest that a chimeric derivative of the pro-resorptive cytokine RANKL can induce bone formation by a mechanism involving commitment to the osteogenic phenotype.

Xu J et al ("Cloning, sequencing, and functional characterization of the rat homologue of receptor activator of NF-kappaB ligand" J. BONE MINER RES. vol. 15, no. 11, 2000, pages 2178 - 2186) investigated truncated domains within the TNF-like core region of rat RANKL that were expressed as glutathione S-transferase (GST) fusion proteins for their ability to induce osteoclastogenesis. Xu et al state that GST-rRANKL (aa160-318) containing the full TNF-like core region had the highest capability to induce the formation of osteoclast-like cells from RAW264.7 cells. GST-rRANKL (aa239-318 and aa160-268) had lesser degrees of osteoclast inductivity.

Meiyanto et al ("Osteoclast differentiation factor modulates cell cycle machinery and causes a delay in s phase progression in RAW264 cells" BIOCHEM. BIOPHYS. RES. COMMUN. vol. 282, no. 1, 2001, pages 278 - 283) state that osteoclast differentiation factor (ODF) induces pleiotropic effects on cell cycle regulatory genes in RAW264 cells during the initial phase of the differentiation process to osteoclasts.

Mizuno et al ("Transgenic mice overexpressing soluble osteoclast differentiation factor (sODF) exhibit severe osteoporosis" J. BONE MINER. METAB. vol. 20, no. 6, 2002, pages 337 - 344) examined osteoclast differentiation factor by generating two types of transgenic mice overexpressing soluble osteoclast differentiation factor (sODF). Mice overexpressing sODF ubiquitously from the early developmental stage died at the late fetal stage, while mice expressing sODF only in the liver after birth exhibited a marked decrease in bone mineral density with aging compared with their non-transgenic littermates, and in addition, the strength of their femurs was extremely reduced. Mizuno et al conclude that excessive production of sODF causes osteoporosis by accelerated osteoclastogenesis.

Kong et al ("OPGL is a key regulator of osteoclastogenesis, lymphocyte development and lymph-node organogenesis" NATURE vol. 397, no. 6717, 1999, pages 315 - 323) disclose that mice with a disrupted opgl gene show severe osteopetrosis and a defect in tooth eruption, and completely lack osteoclasts as a result of an inability of osteoblasts to support osteoclastogenesis and suggest that OPGL is a new regulator of lymph-node organogenesis and lymphocyte development and is an essential osteoclast differentiation factor *in vivo.*

JP 2004 526748 A relates to a method of enhancing bone formation comprising administering an effective amount of 1) an oligomeric complex of one or more of RANKL, a RANKL fusion protein or an analog, derivative or mimic thereof, 2) an osteogenic compound capable of enhancing activity of one or more intracellular proteins in osteoblasts or osteoblast precursors, wherein said activity is indicative of bone formation, or 3) an osteogenic compound capable of inactivating one or more phosphatases in osteoblasts or osteoblast precursors, wherein said inactivation is indicative of bone formation. It is suggested that the method may be used to treat a disease or condition manifested at least in part by the loss of bone mass by administering to a patient a pharmaceutical composition comprising an oligomeric complex or osteogenic compound disclosed herein.

### Disclosure of the Invention

It is an object of the present invention to provide a reagent containing a fused protein of RANKL with GST epitope tag that has improved effects of differentiating and activating osteoclasts compared with the case of using RANKL alone.

The present inventors have found that the activity of soluble RANKL can be enhanced *in vitro* and *in vivo* by fusing soluble RANKL with a different type of protein or peptide used as a tag. This had led to the completion of the present invention.

Specifically, the present invention is described as follows.
1. The use of a fused protein of soluble RANKL with glutathione-S-transferase in which the differentiating and activating function of soluble RANKL is improved, as an active ingredient, for producing a non-human osteopenia animal model.
2. The use according to 1, wherein the agent has interspecies cross-reactivity.
3. An *in vitro* method of differentiating and forming osteoclasts from myelocytes, spleen cells, or peripheral blood cells, comprising culturing myelocytes, spleen cells, or peripheral blood cells collected from an animal in the presence of a fused protein of soluble RANKL with glutathione-S-transferase in which the differentiating and activating function of soluble RANKL is improved.

### Brief Description of the Drawings

Fig. 1A and 1B show images of osteoclast differentiation and formation induced by a fused protein of soluble RANKL with an epitope tag.
Fig. 2 is a graph showing TRAP activity (absorbance) representing the degree of osteoclast differentiation and formation induced by a fused protein of soluble RANKL with an epitope tag.
Figs. 3A and 3B are graphs each showing effects exhibited by a fused protein of soluble RANKL with an epitope tag upon osteoclasts in terms of life-extension (3A) and activation (3B).
Fig. 4 is a graph showing fluctuations in the serum calcium concentration in a case in which a fused protein of soluble RANKL with an epitope tag was administered to mice.
Fig. 5 is a graph showing fluctuations in the serum CTx concentration in a case in which a fused protein of soluble RANKL with an epitope tag was administered to mice.
Fig. 6 is a graph showing fluctuations in the serum TRAP-5b concentration in a case in which a fused protein of soluble RANKL with an epitope tag was administered to mice.
Fig. 7 is a graph showing fluctuations in the serum osteocalcin concentration in a case in which a fused protein of soluble RANKL with an epitope tag was administered to mice.
Fig. 8 is a graph showing fluctuations in the serum ALP concentration in a case in which a fused protein of soluble RANKL with an epitope tag was administered to mice.
Fig. 9A and 9B are graphs each showing fluctuations in femur bone density in a case in which a fused protein of soluble RANKL with an epitope tag was administered to mice.
Fig. 10A is a graph showing TRAP activity (absorbance) representing the degree of osteoclast differentiation and formation induced by a fused protein of soluble RANKL with an epitope tag.
Fig. 10B shows images of osteoclast differentiation and formation induced by a fused protein of soluble RANKL with an epitope tag.
Figs. 11A and 11B are graphs each showing preservation stability of a fused protein of soluble RANKL with an epitope tag. In fig. 11A, the TRAP activity (absorbance) represents the osteoclast differentiation activity before preservation. In fig. 11B, the TRAP activity (absorbance) represents the osteoclast differentiation activity after preservation for 2 months.
Fig. 12 is a chart showing the cross-reactivity of a fused protein of soluble RANKL with an epitope tag. RANKL1 denotes mouse soluble RANKL (produced by Peprotech) and RANKL2 denotes mouse soluble RANKL (produced by R&D).
Fig. 13 shows images of osteoclast formation induced by soluble RANKL and GST-RANKL.

### Best Mode for Carrying Out the Invention

Hereinafter, the present invention is described in greater detail.

The agent for differentiating and activating osteoclasts, the agent for inducing osteopenia in an animal, and the agent for producing an osteopenia animal model of the present disclosure each contain, as an active ingredient, a fused protein of soluble RANKL (sRANKL) with an epitope tag.

RANKL (receptor activator of NF-κB ligand) serves as a ligand for RANK (receptor activator of NF-κB), which is a TNF super family member, and RANKL is a type 2 transmembrane protein having an intracellular domain (a domain comprising amino acids at positions 1 to 48 from the N-terminal of RANK), a transmembrane domain, and an extracellular domain (JP Patent Publication (Kohyo) No. 2002-509430 A and WO98/46644 (JP Patent No. 3523650)). In the extracellular domain, a domain comprising amino acids at position 152 from the N-terminal and the following positions is a TNF ligand family homologous domain. Soluble RANKL does not contain an intracellular domain.

Soluble RANKL includes a soluble RANKL derivative and a soluble RANKL analog. The animal origin of soluble RANKL is not limited, and thus RANKL derived from any animal species, such as human-derived RANKL, mouse-derived RANKL, or rat-derived RANKL, can be used. The full-length nucleotide sequence and the amino acid sequence of human-derived RANKL are represented by SEQ ID NOS: 1 and 2, respectively. A soluble RANKL derivative or a soluble RANKL analog includes a protein comprising a partial sequence of the amino acid sequence of RANKL and having the RANKL activity, such as a truncated protein of RANKL. Preferably, a soluble RANKL derivative comprises a TNF ligand family homologous domain starting from an amino acid at position 152 in the amino acid sequence represented by SEQ ID NO: 2. Examples of a soluble RANKL derivative include a protein having an amino acid sequence comprising amino acids at positions 127 to 317, a protein having an amino acid sequence comprising amino acids at positions 140 to 317, and a protein having an amino acid sequence comprising amino acids at positions 159 to 317. Another example thereof is an RANKL derivative derived from a non-human animal, which has an amino acid sequence corresponding to one of the above partial amino acid sequences of human RANKL. Further, examples of a soluble RANKL derivative or a soluble RANKL analog include: a protein having RANKL activity and comprising an amino acid sequence derived from the amino acid sequence represented by SEQ ID NO: 2 by deletion, substitution, or addition of one or several amino acid(s); and a protein having RANKL activity and comprising an amino acid sequence derived from the amino acid sequence of one of the above proteins each comprising a partial amino acid sequence of RANKL by deletion, substitution, or addition of one or several amino acid(s). Herein, the term "one or several" means 1 to 9, preferably 1 to 5, and more preferably 1 or 2.

An epitope tag that forms a fused protein together with soluble RANKL can be a protein or peptide having a sequence capable of binding to a specific compound such as an antibody. In general, an epitope tag is used for fused protein purification. However, in the present invention, an epitope tag has a function of increasing the activity of soluble RANKL. In addition, such an epitope tag has a function of increasing preservation stability in a soluble RANKL solution during cryopreservation.

Examples of an epitope tag include, but are not limited to: glutathione-S-transferase (GST); polyhistidine comprising 2 to 12, preferably 4 or more, more preferably 4 to 7, and further preferably 5 or 6 histidines; FLAG tag (amino acid sequence DYKDDDDK; SEQ ID NO: 3); Myc tag (amino acid sequence EQKLISEEDL; SEQ ID NO: 4); V5 tag (amino acid sequence GKPIPNPLLGLDST; SEQ ID NO: 5); Xpress tag; HQ tag (amino acid sequence HQHQHQ; SEQ ID NO: 6); HA tag (amino acid sequence YPYDVPDYA; SEQ ID NO: 7); AU1 tag (amino acid sequence DTYRYI; SEQ ID NO: 8); T7 tag (amino acid sequence MASMTGGQQMG; SEQ ID NO: 9); VSV-G tag (amino acid sequence YTDIEMNRLGK; SEQ ID NO: 10); DDDDK tag (amino acid sequence DDDDK; SEQ ID NO: 11); S tag (amino acid sequence KETAAAKFERQHIDSC; SEQ ID NO: 12); CruzTag09 (amino acid sequence MKAEFRRQESDR; SEQ ID NO: 13); CruzTag22 (amino acid sequence MRDALDRLDRLA; SEQ ID NO: 14);CruzTag41 (amino acid sequence MKDGEEYSRAFR; SEQ ID NO: 15); Glu-Glu tag (amino acid sequence EEEEYMPME; SEQ ID NO: 16); Ha.11 tag (amino acid sequence CTPTDVPDYASL; SEQ ID NO: 17); KT3 tag (amino acid sequence PPEPET; SEQ ID NO: 18); thioredoxin; a maltose binding protein (MBP); an immunoglobulin Fc region; and β-galactosidase. Of these, glutathione-S-transferase is preferable.

A fused protein of soluble RANKL with an epitope tag can be obtained by ligating the genes encoding the respective components to each other and causing the expression of the resultant. Fusion of the gene encoding RANKL with the gene encoding an epitope tag can be carried out by a conventional gene recombination method with the introduction of appropriate restriction sites. In such case, it is necessary to exclude a stop codon between the genes to be fused. The distance between the genes to be fused is not limited, and a linker may be contained therebetween. In addition, it is necessary to allow the open reading frames of the two genes to overlap each other. The above epitope tag can be fused either on the N-terminal side or on the C-terminal side of the amino acid sequence of RANKL.

The nucleotide sequence of DNA encoding a fused protein of GST with a protein having an amino acid sequence comprising amino acids at positions 127 to 317 of the amino acid sequence of RANKL and the amino acid sequence of the fused protein are represented by SEQ ID NOS: 19 and 20, respectively. The nucleotide sequence of DNA encoding a fused protein of GST with a protein having an amino acid sequence comprising amino acids at positions 140 to 317 of the amino acid sequence of RANKL and the amino acid sequence of the fused protein are represented by SEQ ID NOS: 21 and 22, respectively. In addition, the nucleotide sequence of DNA encoding a fused protein of GST with a protein having an amino acid sequence comprising amino acids at positions 159 to 317 of the amino acid sequence of RANKL and the amino acid sequence of the fused protein are represented by SEQ ID NOS: 23 and 24, respectively.

The thus produced fused gene is incorporated into an appropriate available expression vector so as to be expressed therein such that a fused protein of interest can be recovered and purified. In addition, the gene can be expressed also in a cell-free system.

Any vector can be used as a vector as long as the vector can be replicated in host cells such as plasmids, phages, and viruses. A vector comprises a replication origin, a selection marker, and a promoter. It may further comprise an enhancer, a transcription termination sequence (terminator), a ribosome binding site, a polyadenylation signal, and the like, according to need. Alternatively, a vector into which a gene encoding an epitope tag such as glutathione-S-transferase has been incorporated in a preliminary step can be used.

DNA can be introduced into a vector by a conventionally known method. Desirably, such a vector comprises: a polylinker containing different restriction sites; or a single restriction site. A specific restriction site in a vector is cleaved with a specific restriction enzyme and DNA can be inserted into the cleavage site. An expression vector containing a fused gene is used for transformation of an appropriate host cell such that a fused protein encoded by the fused gene can be expressed and produced in the host cell.

Examples of a host cell include: bacterial cells of *Escherichia coli*, *Streptomyces, Bacillus subtilis*, and the like; fungal cells; bakers' yeast cells; yeast cells; insect cells; and mammalian cells.

Transformation can be carried out by a conventionally known method such as the calcium chloride method, the calcium phosphate method, DEAE-dextran mediated transfection, electroporation, lipofection, or the like.

The obtained recombinant fusion protein can be purified by a variety of purification methods. For instance, ammonium sulfate precipitation, gel filtration, ion-exchange chromatography, affinity chromatography, and the like can be used alone or in combination according to need. In a case in which an expression product is expressed as a fused protein comprising GST or the like, purification can be carried out based on the characteristics of a protein or peptide fused with a protein of interest. For instance, when a fused protein comprising GST is expressed, GST has an affinity to glutathione and therefore the fused protein can be efficiently purified by affinity chromatography with the use of a column containing glutathione-bound carriers. Also, when a fused protein comprising a histidine tag is expressed, such a protein having a histidine tag binds to a chelate column and therefore the fused protein can be purified with the use of a chelate column. Further, a fused protein comprising an arbitrary epitope tag can be purified by affinity chromatography with the use of an antibody that recognizes an epitope of the epitope tag.

The activity of soluble RANKL can be enhanced not only *in vitro* but also in *vivo* in the case of the fused protein of soluble RANKL with an epitope tag of the present disclosure. Herein, the term "activity of soluble RANKL" refers to *in vitro* activity of forming osteoclasts by cell culture with the use of myelocytes, spleen cells, precursor cells in the peripheral blood, a macrophage cell line, or the like, or *in vitro* activity of promoting life extension or bone resorption potency of purified osteoclasts. Also, the activity refers to *in vivo* activity of increasing the number of osteoclasts so as to activate osteoclasts for promotion of bone resorption. Specifically, increases or decreases in the activity of RANKL can be found based on increases in the number of osteoclasts on the bone surface and in the osteoclast surface area, decreases in bone density and in bone mass, increases in serum bone resorption markers (e.g., calcium, a degraded collagen product (CTx), and tartrate-resistant acid phosphatase (TRAP-5b)), and the like.

The fused protein of soluble RANKL with an epitope tag peptide of the present disclosure induces *in vitro* and *in vivo* osteoclast differentiation, and it further activates and promotes the bone resorption activity of matured osteoclasts. Soluble RANKL alone, to which an epitope tag has not been bound, can also exhibit such effects. However, the fused protein of soluble RANKL with an epitope tag peptide of the present invention has improved osteoclast differentiation and activation potency compared with the case of using RANKL alone. The osteoclast differentiation and activation potency of the fused protein of soluble RANKL with an epitope tag peptide is significantly improved compared with the case of using soluble RANKL alone. For instance, in a case in which such fused protein is added *in vitro* to myelocytes or spleen cells derived from an animal (e.g., a human, a mouse, or a rat), RAW cells, which are mouse macrophage-like cells, or the like, the number and the size of formed osteoclasts increases to a greater extent than in the case of the addition of soluble RANKL alone. In addition, in the above case, the life extension of isolated matured osteoclasts and the improvement in bone resorption potency of osteoclasts can be achieved to a greater extent than in the case of the addition of soluble RANKL alone. Further, the potency inherent in RANKL is enhanced in the fused protein of soluble RANKL with an epitope tag peptide, and thus it exhibits effects of differentiating and activating osteoclasts to an extent that cannot be achieved with the addition of RANKL alone.

When used *in vitro,* the fused protein of soluble RANKL with an epitope tag peptide is added at a concentration of 0.1 nM or more, preferably 0.5 nM or more, more preferably 1 nM or more, further preferably 2 nM or more, even further preferably 2.5 nM or more, and even further preferably 5 nM or more.

In addition, M-CSF may be added when the fused protein of soluble RANKL with an epitope tag is used *in vitro* to induce osteoclast differentiation and activation.

As described above, the fused protein of soluble RANKL with an epitope tag peptide can be used *in vitro* and *in vivo* as an agent for differentiating and activating osteoclasts and thus it can be preferably used for studies of bone metabolism in animals and the like.

In addition, when the fused protein of soluble RANKL with an epitope tag is administered *in vivo* to an animal, the fused protein induces osteoclast differentiation in the body of the animal and further activates and promotes the bone resorption activity of matured osteoclasts. Further, increases in the number of osteoclasts on the bone surface and in the osteoclast surface area, decreases in the bone density and in the bone mass, and increases in serum bone resorption markers are observed in the animal. Such effects are more significantly improved when the fused protein of soluble RANKL with an epitope tag is administered to an animal than when soluble RANKL alone is administered to the same.

Animals to which the fused protein of soluble RANKL with an epitope tag is administered are not limited, and thus it can be administered to all animals such as humans, monkeys, mice, and rats. The fused protein of soluble RANKL with an epitope tag acts on animals irrespective of species differences. Specifically, for instance, a fused protein of human-derived soluble RANKL with an epitope tag acts on RANKs derived from the other animals such as mice, monkeys, and rats, and such a fused protein also exhibits osteoclast induction activity in the other animal species. According to the present disclosure, when a fused protein of soluble RANKL with an epitope tag acts on an animal irrespective of species differences, it can be said that such a fused protein has the cross-reactivity, interspecies cross-reactivity, or effects based on interspecies cross-reactivity.

M-CSF also may be added also in a case in which the above fused protein of soluble RANKL with an epitope tag is administered to an animal.

The amount of the fused protein of soluble RANKL with an epitope tag to be administered to an animal is not limited, and it can be adequately determined depending on animal species. For instance, the fused protein can be administered to mice in an amount of 10 nmol to 5000 nmol and preferably 50 nmol to 1000 nmol per individual mouse. The administration route is not limited and thus the fused protein can be administered in the form of an intravenous injection, an intraperitoneal injection, a subcutaneous injection, a muscular injection, a suppository, an ophthalmic preparation, or the like.

As described above, the fused protein of soluble RANKL with an epitope tag can be used as an agent for inducing osteopenia. Further, an animal with a decrease in bone mass can be used as an osteopenia animal model for screening for a therapeutic or prophilaxic agent for bone diseases characterized by decreases in bone density and bone mass. Examples of bone diseases characterized by decreases in bone density and bone mass include osteoporosis and osteopenia.

In addition, the activity of RANKL is not limited to osteoclast differentiation and activation, and it also includes activity of promoting lymphocyte differentiation, dendritic cell activation, mammary gland epithelial cell differentiation, lymph node formation, and the like. Thus, the fused protein can also be used for a method of inducing promotion of such effects. That is, the present disclosure encompasses a composition selected from the group consisting of an agent for differentiating lymphocytes, a dendritic cell activator, an agent for differentiating mammary gland epithelial cells, and an agent for forming lymph nodes, which contains, as an active ingredient, a fused protein of soluble RANKL with an epitope tag in which at least one function of soluble RANKL selected from the group consisting of a lymphocyte differentiating function, a dendritic cell activating function, a mammary gland epithelial cell differentiating function, and a lymph node forming function has been improved.

The agent for differentiating and activating osteoclasts and the other compositions of the present disclosure may contain a carrier, a diluent, or an excipient which are generally used in the field of drug formulation. Examples of a carrier or an excipient that can be used for tablets include lactose and magnesium stearate. Examples of an injectable aqueous liquid that can be used include physiological saline, glucose, and isotonic solutions containing different adjuvants, which can be used in combination with an appropriate solubilizing adjuvant such as polyalcohol (e.g., alcohol or propylene glycol) or a nonion surfactant activator. Examples of an oily liquid that can be used include sesame oil and soybean oil, which can be used in combination with a solubilizing adjuvant such as benzyl benzoate or benzyl alcohol.

The present invention is hereafter described in greater detail with reference to the following examples, although the technical scope of the present invention is not limited thereto.

### Example 1: Confirmation of in vitro effects of GST-RANKL

### Preparation of GST-RANKL

*Sal*I and *Not*I sites were added to cDNA encoding human RANKL residues 140-317 by PCR. The resultant was cloned downstream of Glutathione S-transferase of pGEX-4T-2 (GE healthcare; Genbank Accession Number: U13854) with the use of the endonucleases. Protein expression was induced in BL21 (DE3) *Escherischia coli* (Invitrogen) with IPTG (final concentration: 0.5 mM). Then, bacterial cells were suspended in an extraction buffer (50 mM Tris-HCl (pH 8.0), 100 mM NaCl, 1 mM EDTA, 1 mM DTT, and 1%(v/v) TritonX-100) and pulverized at 4°C with the use of a sonicator. After centrifugation at 18000 × g for 15 minutes, the supernatant was recovered and applied to a Glutathione Sepharose column. Subsequently, washing with a washing buffer (50 mM Tris-HCl (pH 8.0), 100 mM NaCl, 1 mM DTT, 0.1% (v/v) TritonX-100) was carried out, followed by elution with a Glutathione solution (20 mM reduced glutathione and 50 mM Tris-HCl (pH 8.0)). The molecular weight and purity of purified GST-RANKL were confirmed by SDS-PAGE. The obtained GST-RANKL was subjected to filter filtration. The molecular weight was 47.0 kDa and the purity was 95% or more. In addition, the endotoxin concentration was determined by limulus amebocyte lysate assay and it was confirmed to be less than 1 /µg.

### Osteoclast formation (1)

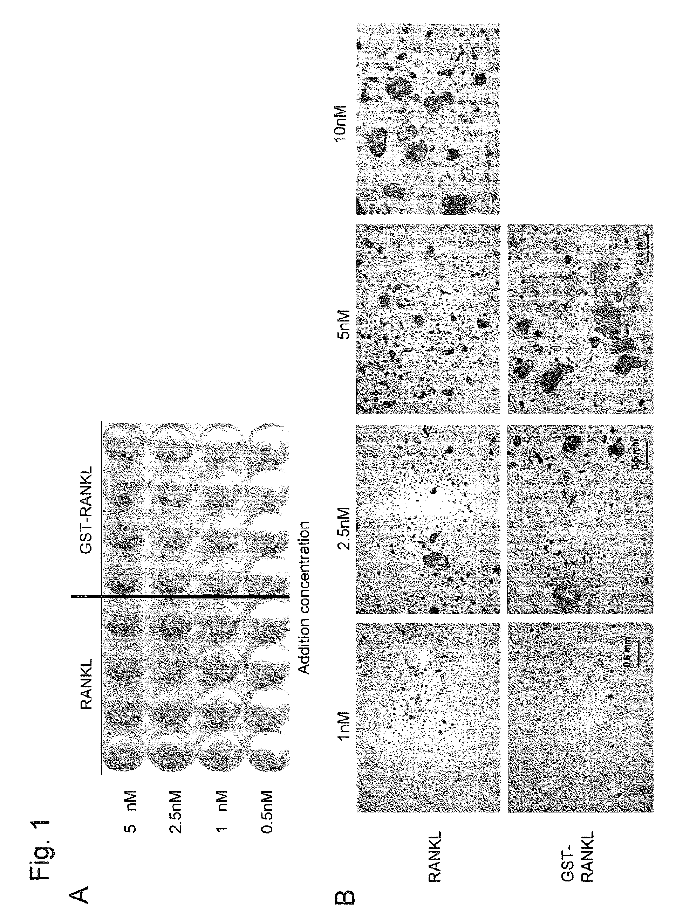

Myelocytes were collected from a 7-week-old ddY mouse and cultured in the presence of M-CSF (10,000 U/mL). Suspension cells were removed and culture was further carried out in the presence of M-CSF for 2 days. Then, the obtained cells were seeded on a 48-well plate at 5 × 10⁵ cells/well. GST-RANKL and soluble RANKL (Peprotech) were separately added to wells at concentrations of 0.5, 1, 2.5, and 5 nM, followed by culture at 37°C in a CO₂ incubator. The supernatant of cells that had been cultured for 3 days was discarded and the cells were fixed with a neutral buffered formalin solution for 2 minutes. Acetone/ethanol (acetone : ethanol = 1:1) was added thereto for refixation for 1 minute. After fixation, acetone/ethanol was removed, followed by drying. After the addition of 500 µl of a substrate solution (NaPHTOL As-MX Phosphate: 0.26 mM; dimethylformamide: 150 mM; TRAP buffer (CH₃COOH: 30 mM; sodium acetate trihydrate: 85 mM; and sodium tartrate: 25 mM); and Fast Red (SIGMA): 2.33 mM), the cells were confirmed to be stained as a result of microscopic observation, followed by washing with water. Osteoclasts were stained in red in a concentration dependent manner (fig. 1A). In addition, GST-RANKL-containing cells were found to be stained to a greater extent than soluble RANKL-containing cells. Further, osteoclasts were microscopically observed. Osteoclasts formed from GST-RANKL-containing cells were found to be larger than those formed from soluble RANKL-containing cells. The concentration of soluble RANKL added was increased to 10 nM and examination was conducted in the manner described above. As a result, formation of osteoclasts larger than those obtained with soluble RANKL at 5 nM was confirmed. However, formation of osteoclasts larger than those formed with cells containing GST-RANKL at 5 nM was not observed (fig. 1B).

GST-RANKL strongly acted on osteoclast formation, and there were clear differences in terms of osteoclast size. Thus, it has been found that addition of GST results in exhibition of strong activity.

### Osteoclast formation (2)

RAW264 cells were seeded on a 96-well plate (2000 cells/well). GST-RANKL and soluble RANKL (Peprotech) were separately added to wells at concentrations of 0.625, 1.25, and 2.5 nM, followed by culture at 37°C in a CO₂ incubator. 3 days later, the cells were further cultured for 1 day in a medium containing GST-RANKL or soluble RANKL at an equivalent concentration. In addition, PBS and GST were added to a control case in the same manner as above for comparison. Each measurement value was verified by ANOVA and Dunnett methods.

The supernatant of cultured RAW264 cells was discarded and fixed for 1 minute with the addition of acetone/ethanol (acetone : ethanol = 1:1) in a volume of 100 µl per well. After fixation, acetone/ethanol was removed, followed by drying. Incubation was carried out at a room temperature for 30 minutes with the addition of 100 µl of a substrate solution (obtained by adding a 50 mM sodium tartrate solution to a buffer (pH 4.5) at a volume ratio of 1:10 (such buffer containing 1.5 g/ml p-nitrophenyl phosphate and 50 mM citric acid and being obtained by mixing sodium citrate dehydrate (3.3 mM) with citric acid monohydrate (50 mM) so as to adjust the pH to 4.5)).

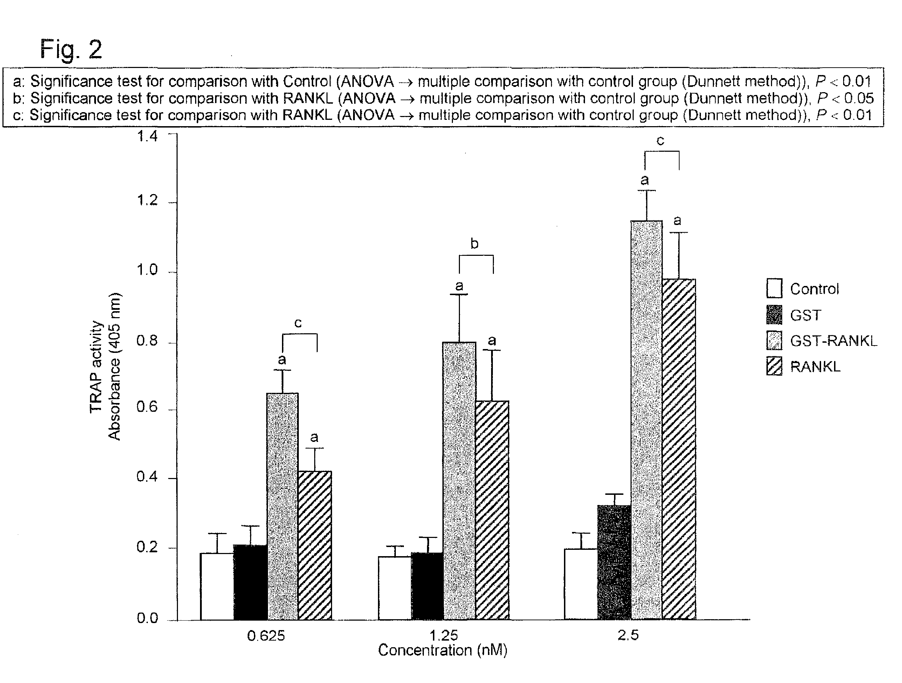

The TRAP activity increased depending on the GST-RANKL and soluble RANKL concentrations. The activity levels of GST-RANKL were significantly higher than those of soluble RANKL at concentrations of 1.25 and 2.5 nM (fig. 2). At such concentrations, the number of osteoclasts increased in a concentration-dependent manner in both cases of GST-RANKL and soluble RANKL.

### Evaluation of the life extension of matured osteoclasts and the bone resorption potency of osteoclasts

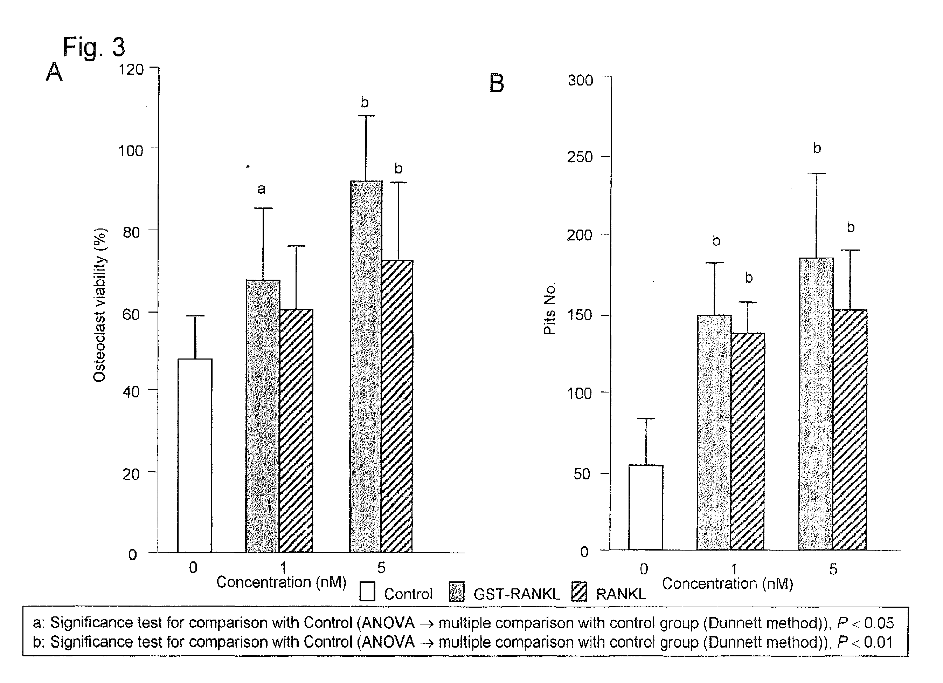

GST-RANKL and soluble RANKL at concentrations of 1 nM and 5 nM were separately added to osteoclasts obtained via coculture of mouse osteoblasts and myelocytes. 0 and 20 hours later, TRAP staining was conducted for the counting of TRAP-positive multinucleated cells. The number of TRAP-positive multinucleated cells determined at 20 hours was divided by the number of cells at 0 hours to calculate the viability (fig. 3A). The cell viability increased in a concentration-dependent manner with the addition of GST-RANKL and soluble RANKL. In the case of GST-RANKL at 5 nM, the viability was approximately twice as high as that for the control group (p<0.01). In addition, GST-RANKL exhibited stronger effects than soluble RANKL.

All cells, including osteoclasts and osteoblasts obtained via coculture of mouse osteoblasts and myelocytes, were seeded on ivory slices, followed by culture for 2 hours. GST-RANKL and soluble RANKL were separately added thereto, followed by culture for 24 hours. After culture, cells were removed from ivory slices and the pit number was counted by HE staining. The number increased depending on the GST-RANKL and soluble RANKL concentrations, indicating that the both substances promoted bone resorption. However, GST-RANKL exhibited stronger effects than RANKL (fig. 3B).

### Example 2: Confirmation of in vivo effects of GST-RANKL

GST-RANKL was prepared in the same manner as in Example 1.

### RANKL administration test

GST-RANKL or soluble RANKL (Peprotech) was administered 3 times via an intraperitoneal route to groups of 7-week-old female C57BL/6N mice (10 individuals each) at doses of 57 nmol (low dose) and 426 nmol (high dose) every 24 hours. Exsanguination was performed 1.5 hours after the third administration. A group to which PBS was administered in the same manner as above was used as a control group for comparison.

The exsanguinated blood was subjected to measurement of serum bone resorption parameters (calcium, CTx (type I collagen-crosslinked C-peptide telopeptide), TRAP-5b) and serum osteogenesis parameters (osteocalcin and alkaline phosphatase (ALP)). Calcium was measured by the OCPC method (WAKO, 272-21801). CTx (Nordic Bioscience Diagnostics), TRAP-5b (IDS Ltd, SB-TR103), and osteocalcin (Biomedical Technologies Inc.) were measured by ELISA. ALP was measured by the Bessey-Lowry method (WAKO, 274-04401).

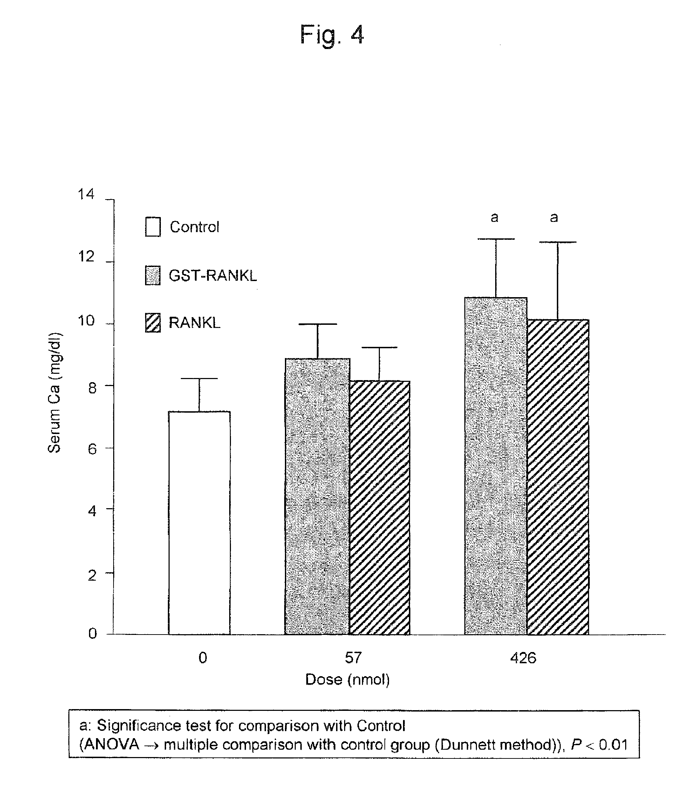

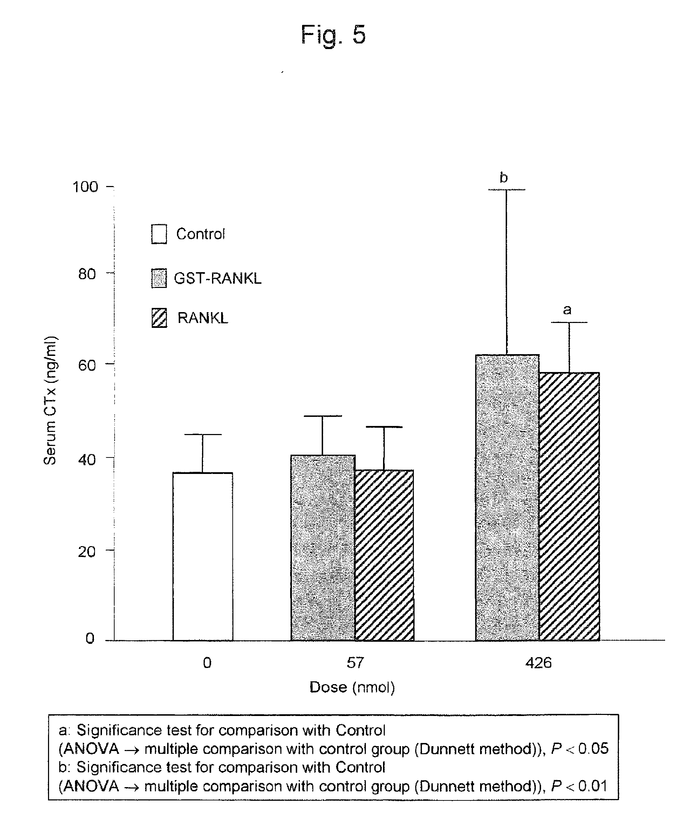

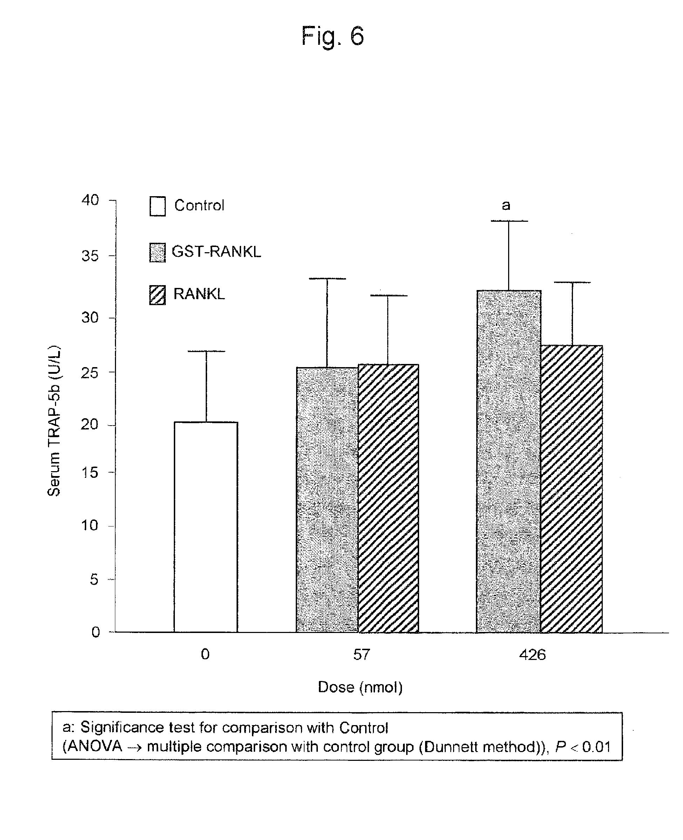

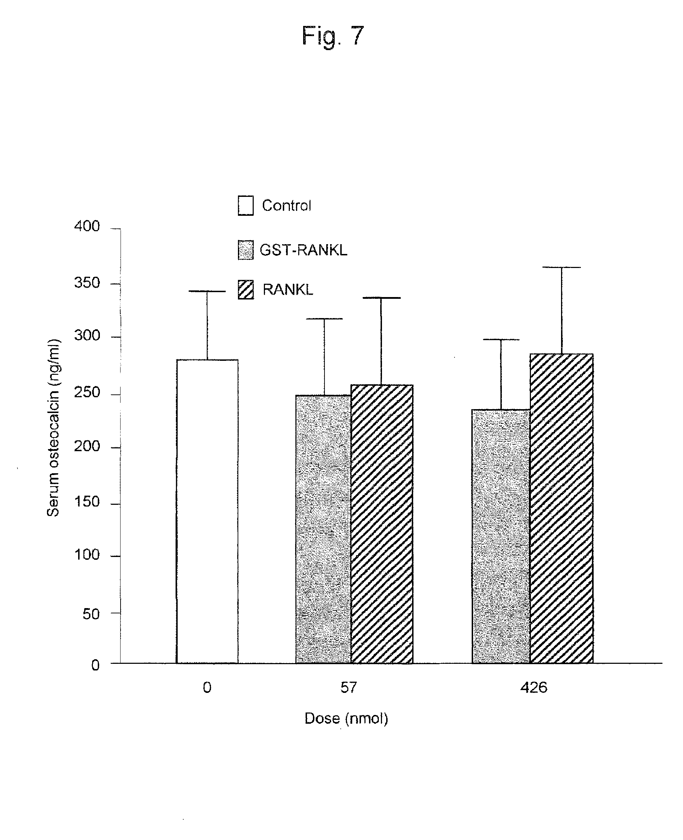

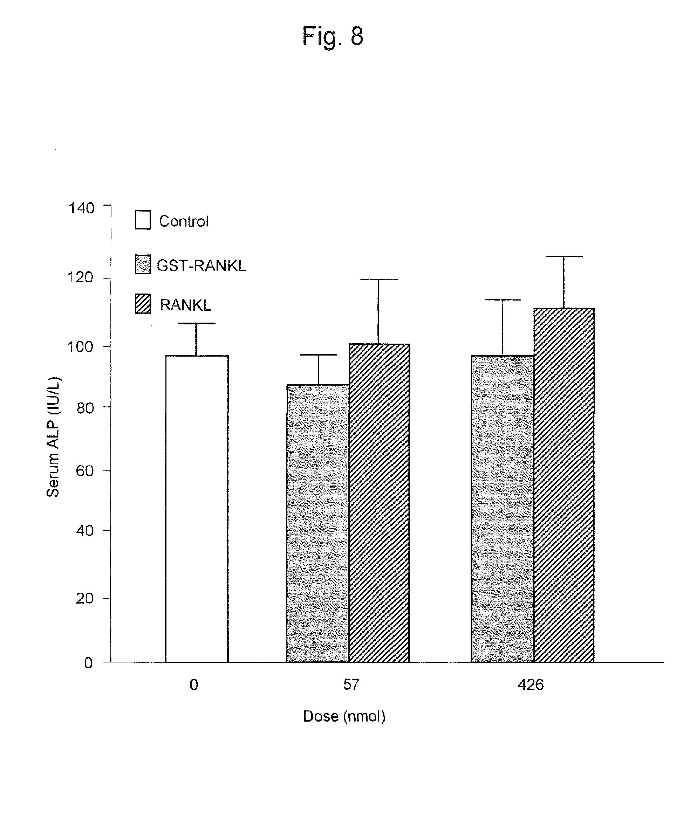

As a result of high-dose administration of either GST-RANKL or soluble RANKL, the serum Ca concentration significantly increased to approximately 1.4-foldas high as that for the control group (p < 0.01) (fig. 4). Also, as a result of high-dose administration, the level of CTx, which is a collagen metabolite, significantly increased to approximately 1.5-fold as high as that for the control group. The significant difference of GST-RANKL was p < 0.01 and that of soluble RANKL was p < 0.05 (fig. 5). In addition, as a result of high-dose administration of GST-RANKL, the level of TRAP-5b significantly increased to approximately 1.5-fold as high as that for the control group (p < 0.01), while soluble RANKL administration did not result in such significant increase (fig. 6). Serum osteocalcin and ALP levels did not change after administration of either GST-RANKL or soluble RANKL or high or low doses (figs. 7 and 8).

### Bone density and bone morphology measurement

The following organs were collected from each exsanguinated mouse: the femur, the tibia, the cerebrum, the lungs, the heart, the liver, the thymus, the spleen, kidneys, and the skin. Naturally occurring lesions were observed by HE staining of the cerebrum, the lungs, the heart, the liver, the thymus, the spleen, the kidneys, and the skin.

Regarding the femur, the cancellous bone was subjected to bone density measurement with the use of pQCT at points 0.6 mm, 0.8 mm, and 1.0 mm away from the growth plate on the proximal side of the cancellous bone.

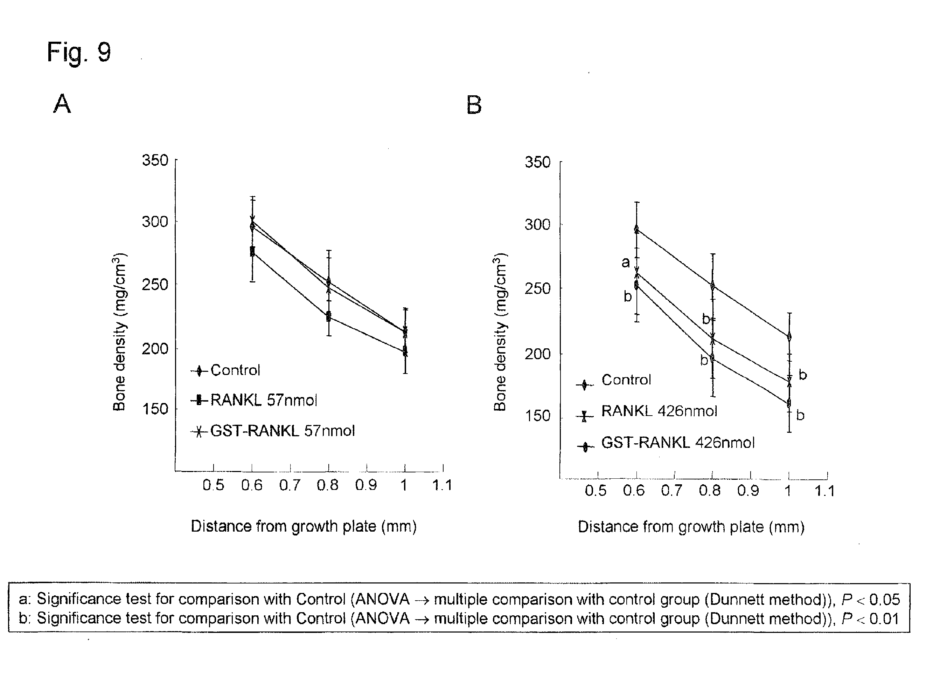

As a result of bone density measurement of the femur with pQCT, the bone density decreased by 10%, 23%, and 30% at the 0.6-, 0.8-, and 1.0-mm points, respectively, in the case of high-dose GST-RANKL administration. Also, the bone density decreased by 10%, 17%, and 20% at the 0.6-, 0.8-, and 1.0-mm points, respectively, in the case of high-dose soluble RANKL administration (figs. 9A and 9B). Verification of the significant difference was carried out by the Anova and Dunnett methods. Accordingly, the significant difference was p < 0.05 for the point 0.6 mm away from the growth plate in the high-dose soluble RANKL administration group. The significant difference was p < 0.01 for the 0.8- and 1.0-mm points in the same group and for each measurement point in the high-dose GST-RANKL administration group (fig. 9B). In addition, no significant difference was obtained in the low-dose GST-RANKL administration group or the low-dose soluble RANKL administration group (fig. 9A).

High-dose GST-RANKL and soluble RANKL administration resulted in osteoclast differentiation, life-extending effects on matured osteoclasts, the improvement of the bone resorption potency of osteoclasts, increases in bone resorption parameters, decreases in bone density and bone mass unit, and an increase in the number of osteoclasts. GST-RANKL exhibited stronger activity than that of soluble RANKL in terms of osteoclast formation ability. The results suggested that GST-fused soluble RANKL exhibits improved activity in the phase of osteoclast differentiation, life extension and activation.

### Example 3

### GST-RANKL activity

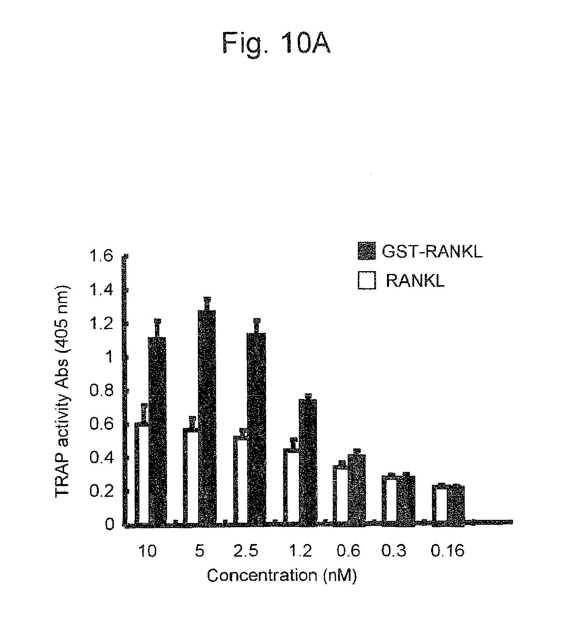

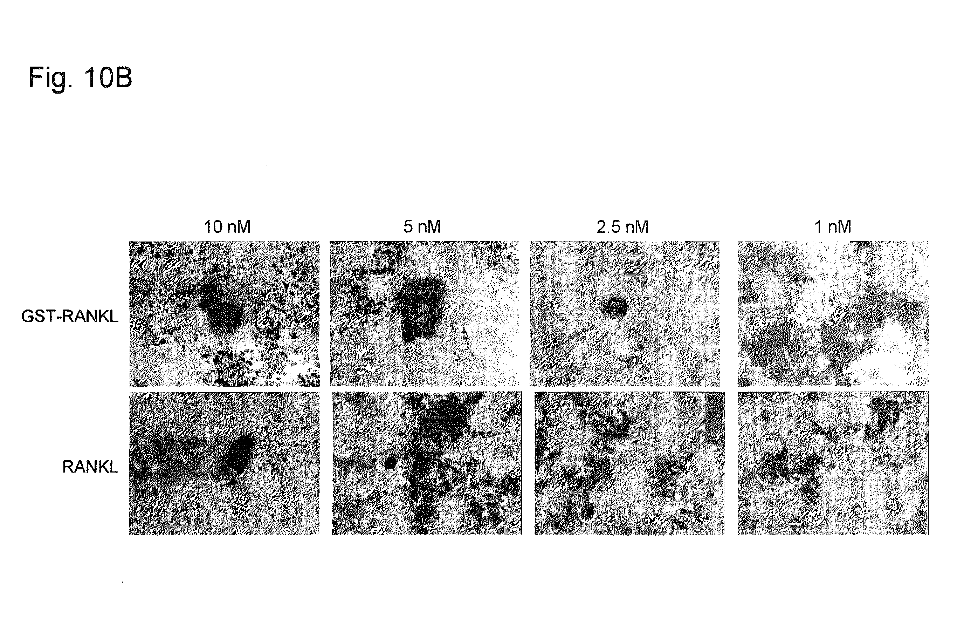

GST-RANKL and soluble RANKL (produced by Peprotech) were separately added to RAW264 cells at serially diluted concentrations of 10, 5, and 2.5 nM. 4 days later, the TRAP activity was determined by the method described in Example 1. The osteoclast differentiation activity after the addition of soluble RANKL (10 nM) was already at the saturation level, and thus the activity did not increase at higher concentrations. As described above, it has been found that soluble RANKL is inferior to GST-RANKL, and that GST-fused RANKL exhibits improved activity and enhanced potency inherent in RANKL such that GST-RANKL exhibits osteoclast differentiation and activation effects to an extent that cannot be achieved with the addition of RANKL alone (fig. 10A). In addition, osteoclasts in the above case were microscopically observed. Accordingly, GST-RANKL was found to induce osteoclasts larger than those induced by soluble RANKL (fig. 10B).

### GST-RANKL preservation stability

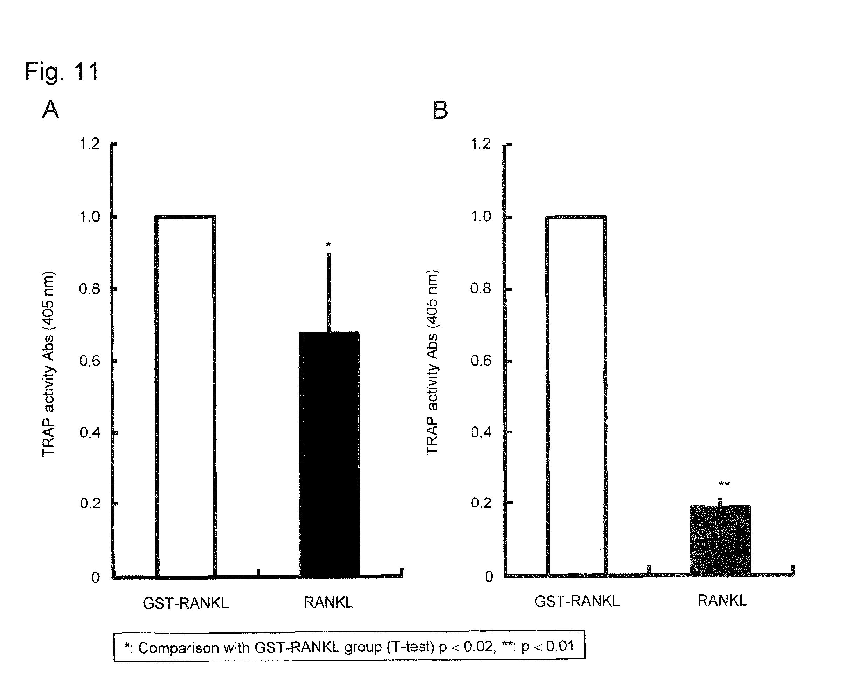

In order to compare the stability of GST-RANKL and that of soluble RANKL, the osteoclast induction activity levels of GST-RANKL and soluble RANKL (produced by Peprotech) (5 nM each) were determined based on the TRAP activity. Thereafter, GST-RANKL and soluble RANKL were preserved at -20°C or lower for 2 months. Then, the osteoclast induction activity was measured in a similar manner. The obtained measurement results were each represented by the proportion of soluble RANKL activity to GST-RANKL activity, which was determined to be 1, provided that both activity levels were measured at the same time. In an experiment in which soluble RANKL, which is a lyophilized product, was dissolved in a solvent and immediately subjected to measurement, the soluble RANKL activity corresponded to approximately 60% of that of GST-RANKL. However, the soluble RANKL activity obtained 2 months thereafter decreased to a level corresponding to approximately 20% of that of GST-RANKL. The results revealed that preservation stability of GST-fused RANKL was obviously improved (figs. 11A and 11B).

### Cross-reactivity of GST-RANKL

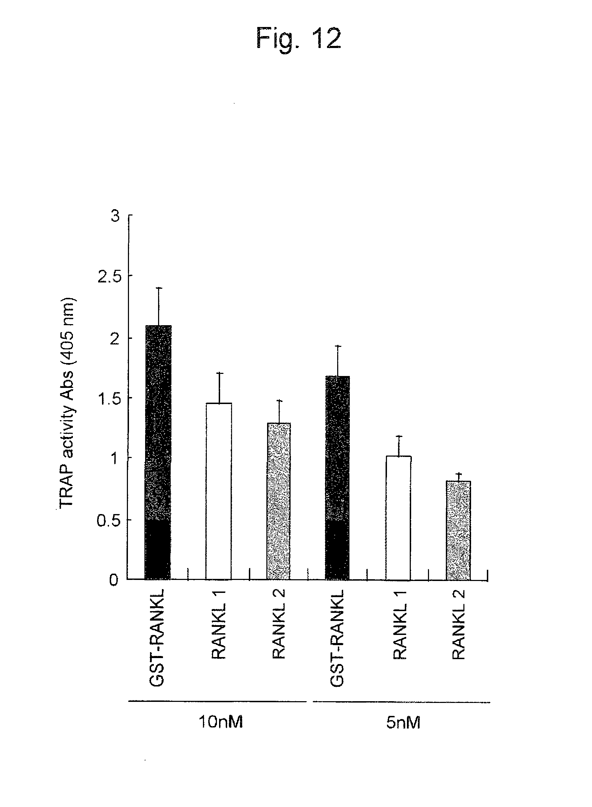

GST-RANKL is one type of human soluble RANKL. However, as a result of comparison with mouse soluble RANKLs (Peprotech and R&D) in terms of osteoclast induction activity (at concentrations of 10 nM and 5 nM), GST-RANKL was found to have activity at least twice as high as that of mouse soluble RANKL, despite the fact that RAW264 cells are mouse-derived cells (fig. 12). The results revealed that GST-fused human soluble RANKL (GST-RANKL) exhibits stronger osteoclast induction activity in RAW264 cells (having mouse RANKs serving as RANKL receptors) than mouse soluble RANKL. The results also indicate that GST-fused human soluble RANKL acts on mouse RANK in a way that transcends species differences, and that the effects thereof are stronger than those obtained by the mouse ligand-receptor reaction between mouse soluble RANKL and mouse RANK.

### Example 4

### Osteoclast differentiation

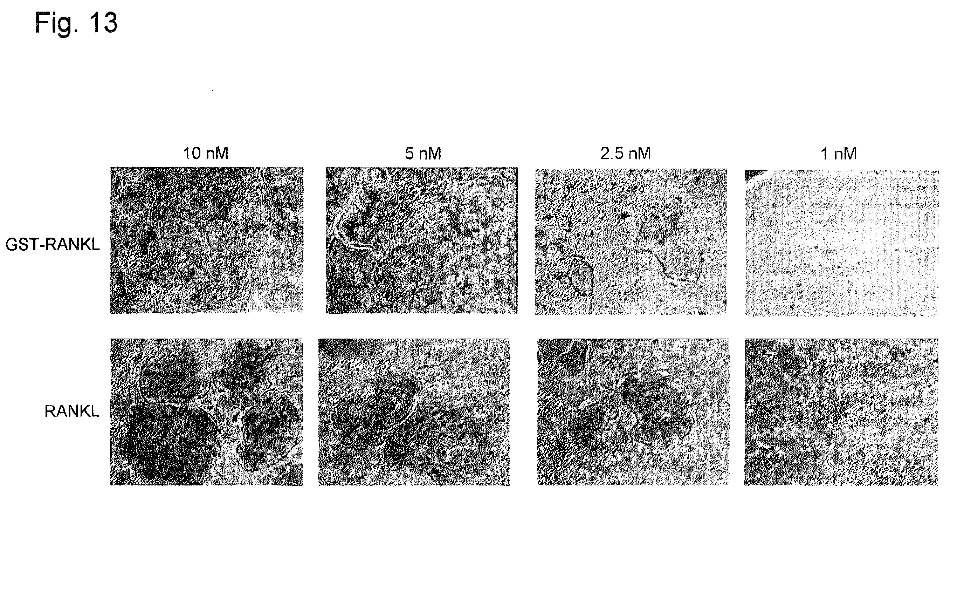

Myelocytes were collected from a 7-week-old ddY mouse and cultured in the presence of M-CSF (10,000 U/mL). Suspension cells were removed and culture was further carried out in the presence of M-CSF for 2 days. Then, the cells were seeded on a 48-well plate at 5 × 10⁵ cells/well. GST-RANKL and soluble RANKL (Peprotech) were separately added to wells at concentrations of 1, 2.5, 5, and 10 nM, followed by culture at 37°C in a CO₂ incubator. The supernatant of cells that had been cultured for 3 days was discarded and the cells were fixed with a neutral buffered formalin solution for 2 minutes. Acetone/ethanol (acetone : ethanol = 1:1) was added thereto for refixation for 1 minute. After fixation, acetone/ethanol was removed, followed by drying. After the addition of 500 µl of a substrate solution (NaPHTOL As-MX Phosphate: 0.26 mM; dimethylformamide: 150 mM; TRAP buffer (CH₃COOH: 30 mM; sodium acetate trihydrate: 85 mM; and sodium tartrate: 25 mM); and Fast Red (SIGMA): 2.33 mM), the cells were confirmed to be stained as a result of microscopic observation, followed by washing with water. Osteoclasts were stained in red in a concentration dependent manner (fig. 13). In addition, GST-RANKL-containing cells were found to be stained more than soluble RANKL-containing cells. Further, osteoclasts were microscopically observed. Osteoclasts formed from GST-RANKL-containing cells were found to be larger than those formed from soluble RANKL-containing cells (fig. 13).

GST-RANKL strongly acted on osteoclast formation and there were clear differences in terms of osteoclast size. Thus, it has been found that addition of GST results in exhibition of strong activity.

### Industrial Applicability

Production of a fused protein of RANKL with an epitope tag, such as GST-RANKL, results not only in the improvement of the specific activity of soluble RANKL but also in the enhancement of the potency thereof. That is, in an *in vitro* osteoclast formation system, an RANKL fused protein such as GST-RANKL can cause formation of a greater number of much larger osteoclasts than those formed by increasing the soluble RANKL concentration to the saturation level. This indicates that the potency of such fused protein is improved in terms of the number of osteoclasts that can be formed and activated, in addition to the specific activity that can be increased. Accordingly, it has become possible to allow RANKL to exhibit strong activity not only *in vitro* but also *in vivo*. A fused protein of RANKL with an epitope tag can be preferably used as a reagent used for bone metabolism studies. Further, when it is administered *in vivo* to an animal, the bone density and the bone mass of the animal decrease. Therefore, the fused protein can be used for production of osteopenia animal models. Further, the activity of RANKL is not limited to osteoclast differentiation and activation, and it also includes an activity of promoting lymphocyte differentiation, dendritic cell activation, mammary gland epithelial cell differentiation, lymph node formation, and the like.

A fused protein of RANKL with an epitope tag can be preferably used as a reagent used for bone metabolism studies. Further, when it is administered *in vivo* to an animal, the bone density and the bone mass of the animal decrease. Therefore, the fused protein can be used for production of osteopenia animal models.

Further, the activity of RANKL is not limited to osteoclast differentiation and activation, and it also includes an activity of promoting lymphocyte differentiation, dendritic cell activation, mammary gland epithelial cell differentiation, lymph node formation, and the like. Therefore, the present disclosure also encompasses the use of the above fused protein as a reagent capable of inducing promotion of such effects.

## Claims

1. The use of a fused protein of soluble RANKL with glutathione-S-transferase in which the differentiating and activating function of soluble RANKL is improved, as an active ingredient, for producing a non-human osteopenia animal model.

2. The use according to claim 1, wherein the agent has interspecies cross-reactivity.

3. An in vitro method of differentiating and forming osteoclasts from myelocytes, spleen cells, or peripheral blood cells, comprising culturing myelocytes, spleen cells, or peripheral blood cells collected from an animal in the presence of a fused protein of soluble RANKL with glutathione-S-transferase in which the differentiating and activating function of soluble RANKL is improved.

## Patentansprüche

1. Verwendung eines Fusionsproteins von löslichem RANKL mit Glutathion-S-Transferase, in dem die differenzierende und aktivierende Funktion von löslichem RANKL verbessert ist, als Wirkstoff zur Bereitstellung eines nichthumanen Osteopenie-Tiermodells.

2. Verwendung gemäß Anspruch 1, wobei das Mittel Interspezies-Kreuzreaktivität (interspecies cross-reactivity) aufweist.

3. In vitro-Verfahren zur Differenzierung und Bildung von Osteoklasten aus Myelozyten, Milzzellen oder peripheren Blutzellen, umfassend Kultivieren von Myelozyten, Milzzellen oder peripheren Blutzellen, welche von einem Tier gewonnen werden, in Gegenwart eines Fusionsproteins von löslichem RANKL mit Glutathion-S-Transferase, in dem die differenzierende und aktivierende Funktion von löslichem RANKL verbessert ist.

## Revendications

1. Utilisation d'une protéine de fusion de RANKL soluble avec la glutathion-S-transférase où la fonction de différenciation et d'activation de RANKL soluble est améliorée, en tant qu'ingrédient actif, pour produire un modèle animal non humain d'ostéopénie.

2. Utilisation selon la revendication 1, dans laquelle l'agent a une réactivité croisée inter-espèces.

3. Procédé in vitro de différenciation et de formation d'ostéoclastes à partir de myélocytes, de cellules spléniques ou de cellules de sang périphérique, comprenant la culture des myélocytes, des cellules spléniques, ou des cellules de sang périphérique récupéré(e)s d'un animal en présence d'une protéine de fusion de RANKL soluble avec la glutathion-S-transférase où la fonction de différenciation et d'activation de RANKL soluble est améliorée.
